(19) Europäisches Patentamt
European Patent Office
Office européen des brevets

(11) Publication number: 0 254 677 A1

# (12) EUROPEAN PATENT APPLICATION

(21) Application number: **87810381.1**

(22) Date of filing: **02.07.87**

(51) Int. Cl.[4]: **C 07 C 87/29**
C 07 C 87/457, C 07 C 93/14,
C 07 D 307/81,
C 07 D 333/58, C 07 F 7/08,
A 61 K 31/135, A 61 K 31/33,
A 01 N 33/04, A 01 N 43/00

(30) Priority: **08.07.86 DE 3622882**

(43) Date of publication of application:
**27.01.88 Bulletin 88/04**

(84) Designated Contracting States:
**BE CH DE FR GB IT LI NL**

(71) Applicant: **SANDOZ AG**
**Lichtstrasse 35**
**CH-4002 Basel (CH)**

(84) Designated Contracting States:
**BE CH FR GB IT LI NL**

(71) Applicant: **SANDOZ-PATENT-GMBH**
**Humboldtstrasse 3**
**D-7850 Lörrach (DE)**

(84) Designated Contracting States: **DE**

(72) Inventor: **Stütz, Anton**
**Donaustrasse 113/7**
**A-2344 Maria Enzersdorf (AT)**

**Heck, Reinhard**
**Wiesenstrasse 29**
**D-6836 Oftersheim (DE)**

The title of the invention has been amended (Guidelines for Examination in the EPO, A-III, 7.3).

(54) **Antimycotic 6-phenyl-2-hexen-4-ynamines.**

(57) The invention provides novel allylamines of formula I

$$R_2 - \underset{R_3}{\overset{R_1}{C}} - \overset{R_4}{N} - CH_2 - CH = CH - C \equiv C - R_5 \qquad I$$

wherein $R_1$ represents a naphtyl, a benzocycloalkyl or a heteroring condensed with a benzen ring, $R_2$, $R_3$ and $R_4$ are hydrogen or an alkyl group and $R_5$ is a benzyl or a phenylsilyl group, processes for their production, pharmaceutical and agricultural compositions containing them and their use as pharmaceuticals or in combatting phytopathogenic fungi.

EP 0 254 677 A1

Bundesdruckerei Berlin

**Description**

## IMPROVEMENTS IN OR RELATING TO ORGANIC COMPOUNDS

The invention relates to novel allylamine compounds, processes for their production, pharmaceutical and agricultural compositions containing them and their use as pharmaceuticals or in combatting phytopathogenic fungi.

Allylamine compounds and their use as antimycotics are known from i.a. EPA 24587. Homopropargylamines having chemotherapeutical and agricultural use are disclosed in EPA I9I,269.

Objective of the present invention is to provide allylamine compounds having advantageous antimycotic properties and/or being particularly useful in combatting phytopatogenic fungi. Another objective of the invention is to provide antifungal compounds suitable for seed treatment.

The invention provides compounds of formula I

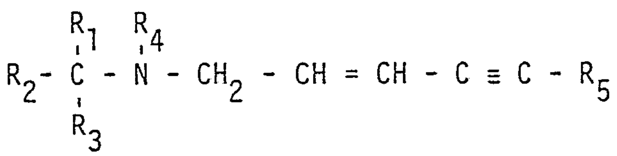 I

wherein $R_1$ is one of the groups of the formula IIa, IIb and IIc,

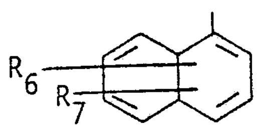

IIa

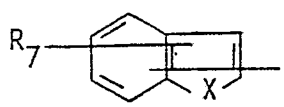

IIb

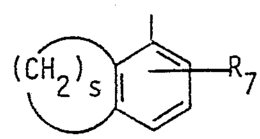

IIc

in which $R_6$ and $R_7$ independently are H, halogen, $CF_3$, $C_{1-5}$alkyl or $C_{1-5}$alkoxy,
X is O, S, O-$CH_2$-, S-$CH_2$-, $CH_2$ or $NR_8$, in which $R_8$ is H or $C_{1-5}$alkyl,
s is 3, 4 or 5,
$R_2$ is H or $C_{1-5}$alkyl,
either $R_3$ and $R_4$ are independently H or $C_{1-5}$alkyl,
or $R_3$ and $R_4$ together $(CH_2)_u$in which u is 3, 4 or 5,
and $R_5$ is a group of formula IIIa or IIIb

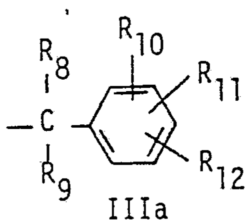

IIIa

or

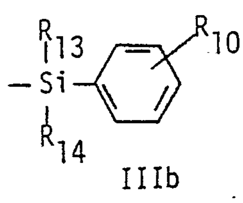

IIIb

wherein $R_8$ and $R_9$ independently are H, $C_{1-5}$alkyl, $C_{1-5}$alkoxy or $CF_3$,
$R_{10}$, $R_{11}$ and $R_{12}$ independently are H, $C_{1-5}$alkyl, $C_{1-5}$alkoxy, $CF_3$, halogen or phenyl,
$R_{13}$ and $R_{14}$ independently are $C_{1-5}$alkyl.

The compounds of formula I are prepared by

a) reacting a compound of formula IV

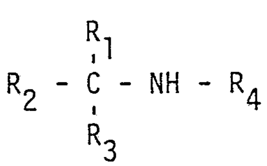 IV

wherein $R_1$ to $R_4$ are defined above,

with a compound of formula V

$A-CH_2-CH=CH-C\equiv C-R_5$ V

wherein $R_5$ is as defined above,

and A is a leaving group,

or b) obtaining compounds of formula Ia

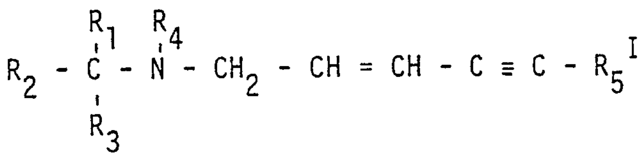 Ia

wherein $R_1$ to $R_4$ are as defined above,

and $R_5{}^I$ is a group of formula IIIa, defined above,

by substituting in a compound of formula VI

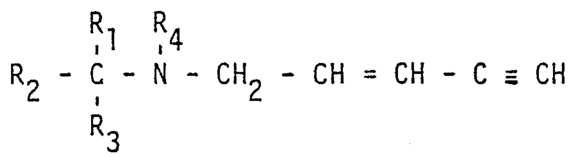 VI

wherein $R_1$ to $R_4$ are as defined above,

the acetylenic hydrogen by an alkali metal and reacting the thus obtained alkali metal salt of the compound of formula VI with a compound of formula IIIc

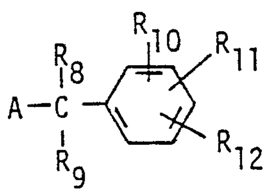 IIIc

wherein $R_8$ to $R_{12}$ and A are as defined above.

Process a) is conveniently carried out in a solvent which is inert under the reaction conditions, e.g. in a lower alcohol such as ethanol, if desired in admixture with water, in an aromatic hydrocarbon such as benzene or toluene, in a cyclic ether such as dioxane or in a dialkylamide of a carboxylic acid such as dimethylformamide. The reaction temperature is advantageously selected between room temperature and boiling temperature of the reaction mixture, and is preferably at room temperature. The reaction is advantageously effected in the present of an acid binding agent, e.g. an alkalimetalcarbonate such as $Na_2CO_3$.

The substitution of the acetylenic hydrogen in process b) may be effected by reacting a compound of formula VI with an organometal compound, e.g. an alkyllithium such as butyllithium. Such substitution reaction is conveniently carried out in a solvent which is inert under the reaction conditions, e.g. a cyclic ether such as tetrahydrofuran, in the absence of water and preferably at a low temperature.

The subsequent alkylation is for example effected with a compound of formula IIIc wherein the leaving group is a halogen. Such reaction may be carried out under the conditions known for the alkylation of a metal salt of acetylene, e.g. in the presence of an aluminium-halogen compound such an $AlCl_3$, $(CH_3)_2AlCl$ or $(C_2H_5)_2$-AlCl and in a solvent which is inert under the reaction conditions, e.g. the chlorinated hydrocarbon, such as l,2-dichloroethane.

Alkyl and alkoxy substituents in the compounds of formula I comprise preferably l to 4, more particularly l or 2 carbon atoms.

Compounds of formula I wherein $R_5$ is phenylalkyl are generically embraced by the scope of EPA 24587, but not specifically disclosed therein. The compounds of the invention have particularly advantageous antimycotic properties.

In the compounds of formula V and IIIc A is for example halogen, particularly Cl or Br, or an organic sulfonyloxy group with l to lO carbon atoms, e.g. an alkylsulfonyloxy group, particularly of l to 4 carbon atoms, such as methylsulfonyloxy, or an alkylphenylsulfonyloxy group, particularly of 7 to lO carbon atoms, such as tosyloxy.

The compounds of formula V are partly novel. They may be obtained according to the following reaction scheme:

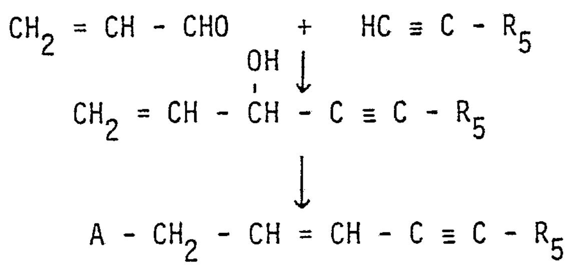

The starting materials are either known, or may be obtained in a manner known per se or analogous to methods disclosed herein.

The compounds of formula I exhibit chemotherapeutic activity. In particular, they exhibit antimycotic activity, as indicated by in vitro tests in various families and types of mycetes, including Trichophyton spp., Aspergillus spp., Microsporum spp., Sporotrix schenkii and Candida spp., at concentrations of, for example 0.003 to 50 microgram/ml, and in vivo in the experimental skin mycosis model in guinea pigs. In the latter test, the test substance is administered daily for 7 days beginning 24 hours after the infection on local application by rubbing the test substance (taken up in polyethylene glycol) on the skin surface. The Candida activity is shown in vivo employing conventional intravaginal/intrauterine- or disseminated-infection models on mice or rats. The activity is shown on local application at concentrations of for example 0.01 to 0.2%. The oral activity is shown in vivo in the guinea-pig-trichophytosis model at dosages of, for example, 2 to 70 mg/kg.

The compounds are therefore indicated for use as antimycotic agents, in particular against dermatophytes.

The compounds may be used in free base form or in the form of chemotherapeutically acceptable acid addition salts. Such salt forms exhibit the same order of activity as the free base forms. Suitable such salt forms are e.g. hydrochloride, hydrogen fumarate or naphthaline-1,5-disulphonate. The compounds may be employed in a manner analogous to that known for compounds having the same chemotherapeutical use, such as griseofulvine, and may be administered parenterally, topically, intravenously or topically. The appropriate daily dosage will depend on various factors, e.g. on the particular compound employed, the treatment desired the mode of administration etc. An indicated daily dose for oral use is from 70 to 2000 mg. If desired this may be administered in divided doses 2 to 4 times a day in unit dosage form containing from about 17.5 mg to about 1000mg or in sustained released form.

It has for example been established that preferred compound (E)-N-methyl-N-(1-naphthylmethyl)-6-methyl-6-phenyl-2-hepten-4-ynamine has a curative dosage (i.e. the dosage at which all guinea pigs infested with Trichophyton mentagraphytes var. quinckeanum 158 are mycologically cured) of 9 × 6 mg/kg, compared with 9 × 70 mg/kg for griseofulvine in the same test.

It is accordingly indicated that the compounds of formula I may be employed at dosages similar or inferior to those normally employed for griseofulvin.

The compounds may be admixed with conventional chemotherapeutically acceptable diluents and carriers such as lactose, starch, talc, magnesium stearate and, optionally, other excipients. The amount or concentration will, of course, vary depending on the compound employed, the treatment desired, the nature of the form etc.

With topical application forms satisfactory results will in general, be obtained when applied at concentration of from 0.05 to 5.0, in particular 0.1 to 1 % by weight.

Accordingly, the invention therefore also concerns a method of treating diseases or infections caused by mycetes which comprises administering to a subject in need of such treatment an effective amount of a compound of formula I or a chemotherapeutically acceptable acid addition salt thereof. It also relates to compounds of formula I or chemotherapeutically acceptable acid addition salts thereof for use as chemotherapeutic agents especially as antimycotics.

The compounds of formula I in free base form or in agriculturally acceptable acid addition salt form (hereinafter agrichemicals of the invention) are useful for combatting phytopathogenic fungi. The interesting fungicidal activity is i.a. observed in in vivo tests against rusts, such as Uromyces appendiculatus on runner beans and Puccinia on wheat, against powdery mildews, such as Erysiphe cichoracearum cuccumber, E. Graminis on wheat, Podosphaera leveotricha on apple and Uncinula necator on grapevine and against other important diseases caused by Septoria in wheat, Helminthosporium in barley etc. at test concentrations of from 0.5 to 500 ppm. The test results also indicate a good plant tolerance.

The agrichemicals of the invention are therefore indicated for use in combatting phytopathogenic fungi, e.g. Basidiomycetes of the order Uredinales such as Puccinia spp., Hemileia spp., Uromyces spp. and Ascomycetes of the order Erysiphales, such as Erysiphe spp., Podosphaera spp., Uncinula spp., Cochliobulus (Helminthosporium) spp. and Leptosphaeria (Septoria) spp.

The amount of agrichemicals of the invention to be applied will depend on various factors such as the compound employed, the subject of the treatment (plant, soil, seed), the type of treatment (e.g. spraying, dusting, dressing), the purpose of the treatment (prophylactic or therapeutic), the type of fungi to be treated

and the application time.

In general, satisfactory results are obtained, if the agrichemicals of the invention are applied in an amount of from about 0.005 to 2.0, preferably about 0.01 to 1 kg/ha, in the case of a plant or soil treatment; e.g. 0.04 to 0.125 kg of active ingredient (a.i.) per ha in crops such as cereals, or concentrations of 1 to 5 g of a.i. per hl in crops such as fruits, vineyards and vegetables (at an application volume of from 300 to 1000 litres/ha - depending on the size of leaf volume of the crop - which is equivalent to an application rate of approximately 10-50 g/ha). The treatment can, if desired, be repeated, e.g. at intervals of 8 to 30 days.

Where the agrichemicals of the invention are used for seed treatment, satisfactory results are in general obtained, if the compounds are used in an amount of from about 0.01 to 10 g per kg of seed, particularly 0.05 to 0.5, preferably about 0.1 to 0.3 g/kg seeds.

In the treatment of soil, active compound concentrations of 0.00001 to 0.1 % by weight, preferably 0.0001 to 0.02 % are desirable at the place of action.

The agrichemicals of the invention in agriculturally acceptable salt form, exhibit in general the same order of activity as the corresponding compounds in free base form. The agrichemicals of the invention are particularly suitable for use in seed treatment. The (E)isomer of Example 1 and the compound of Example 7 show very interesting antifungal activity.

The term soil as used herein is intended to embrace any conventional growing medium, whether natural or artificial.

The compounds of the invention may be used in a great number of crops, such as soybean, coffee, ornamentals (i.a. pelargonium, roses), vegetables (e.g. peas, cucumber, celery, tomato and bean plants), sugarbeet, sugarcane, peanuts, cotton, flax, maize (corn), vineyards, fruits (e.g. apples, pears, prunes, bananas) and in cereals (e.g. wheat, oats, barley, rice).

The invention therefore provides a method of combatting phytopathogenic fungi, comprising applying to the fungi or their locus a fungicidally effective amount of a compound of formula I in free base form or in agriculturally acceptable acid addition salt form.

The invention also provides fungicidal compositions comprising as fungicide a compound of formula I in free base form or in agriculturally acceptable acid addition salt form in association with agriculturally acceptable diluent (hereinafter diluent) and, optionally, additional excipients such as surfactants. In general, such compositions comprise 0.0005 to 90, e.g. 0.001 to 70 percent by weight of active ingredient.

For distribution purposes the compounds will normally be formulated in concentrated form, comprising e.g. from 2 to 90, e.g. 5 to 70% by weight of active ingredient.

The term diluents as used herein means liquid or solid, agriculturally acceptable material, which may be added to the active agent to bring it in an easier or better applicable form, resp. to dilute the active agent to a usable or desirable strength of activity. Examples of such diluents are talc, kaolin, diatomaceous earth, xylene or water.

Such formulations, especially these used in spray form, such as water dispersible concentrates or wettable powders, may contain surfactants (e.g. up to 20 % by weight) such as wetting and dispersing agents, e.g. the condensation product of formaldehyde with naphthalene sulphonate, an alkylarylsulphonate, a lignin sulphonate, a fatty alkyl sulphate, an ethoxylated alkyl phenol and an ethoxylated fatty alcohol.

In addition to the usual diluents and surfactants, the compositions of the invention may comprise further additives with special purposes, e.g. stabilizers, desactivators (for solid formulations or carriers with an active surface), agents for improving the adhesion to plants, corrosion inhibitors, anti-foaming agents and colorants. Moreover, further fungicides with similar or complementary fungicidal activity, e.g. sulphur, chlorothalonil, dithiocarbamates such as mancozeb, maneb, zineb, propineb, trichloromethane-sulphenylphthalimides and analogues such as captan, captafol and folpet, benzimidazoles such as benomyl and carbendazim, or other beneficially-acting materials, such as insecticides may be present in the formulations.

Examples of plant fungicide formulations are as follows (parts are by weight):

**a. Wettable Powder Formulation**

10 Parts of a compound of formula I are mixed and milled with 4 parts ofsynthetic fine silica, 3 parts of sodium lauryl sulphate, 7 parts of sodium lignin sulphonate and 66 parts of finely divided kaolin and 10 parts of diatomaceous earth until the mean particle size is about 5 micron. The resulting wettable powder is diluted with water before use to spray liquor.

**b. Granules**

Onto 94.5 parts by weight of quartz sand in a tumbler mixer are sprayed 0.5 parts by weight of a binder (non-ionic tenside) and the whole thoroughly mixed. 5 parts by weight of a compound of formula I are then added and thorough mixing continued to obtain a granulate formulation with a particle size in the range of from 0.3 to 0.7 mm.

**c. Emulsion Concentrate**

25 Parts by weight of a compound of formula I are mixed with 10 parts by weight of an emulsifier and 65 parts by weight of xylene. The concentrate is diluted with water to the desired concentration.

**d. Seed Dressing**

45 Parts of a compound of the invention are mixed with l.5 parts of diamyl phenoldecaglycolether ethylene oxide adduct, 2 parts of spindle oil, 5l parts of fine talcum and O.5 parts of colorant rhodamin B. The mixture is ground in a contraplex mill at lO,OOO rpm until an average particle size of less than 2O microns is obtained. The resulting dry powder has good adherance and may be applied to seeds, e.g. by mixing for 2 to 5 minutes in a slowly turning vessel.

A preferred subgroup of the compounds of formula I has the following features:

$R_1$ is the group of formula IIa,

$R_2$ and $R_3$ are H,

$R_4$ is $C_{1-4}$alkyl, $R_5$ is the group of formula IIIa in which $R_8$ and $R_9$ are H or $C_{1-4}$alkyl.

Particularly preferred are compounds of formula I, wherein $R_8$ and $R_9$ are $C_{1-4}$alkyl, especially those wherein $R_8$ and $R_9$ are $CH_3$.

$R_{10}$ is preferably in the para-position. Preferred significances of $R_{10}$ are H, halogen, $C_{1-4}$alkoxy, $C_{1-4}$alkyl or phenyl. Where $R_{10}$ is halogen it is preferably F.

$R_{11}$ and $R_{12}$ are preferably H or $C_{1-4}$alkyl.

The following examples illustrate the invention. Temperatures are in Centigrade.

Example l: (E)-N-Methyl-(l-naphthylmethyl)-6-methyl-6-phenyl-2-hepten-4-ynamine and (Z)-N-Methyl-N-(l-naphthylmethyl)-6-methyl-6-phenyl-2-hepten-ynamine (process a)

To a mixture of 0.85 g of N-methyl-N-(n-naphthylmethyl)-amine, 0.53g of $Na_2CO_3$ and 5 ml of dimethylformamide are added l.3 g of l-bromo-6-methyl-6-phenyl-2-hepten-4-yne (a crude cis/trans mixtures. The mixture is stirred over night.

The solvent is separated off in vacuo and the residue distributed between water and ether. The organic phase is separated, dried and concentrated in vacuo. The residue is purified by chromatography on silica gel employing $CH_2Cl_2$/ethylacetate (9:l) as eluant. The (E)-isomer is obtained first, followed by the (Z)-isomer. Both are oils.

Example 2

To a solution of l.l8g of (E)-N-methyl-N-(l-naphthylmethyl)-2-penten-4-inamine in 5 ml hexane are added at -30° under a blanket of Argon 3.3 ml n-butyllithium. The mixture is stirred for l5 minutes and then 5 ml dimethylaluminiumchloride in hexane are added. After 20 minutes 40 ml l,2-dichloroethane are added until the suspension is almost clear. Thereafter 0.95 g of 2-chloro-2-(4-chlorophenyl)propane and an additional 7.5 ml of dimethylaluminiumchloride are added. The cooling bath is taken away and the reaction stirred for 30 minutes at room temperature. The reaction mixture is washed with 2N $N_2OH$ and the organic phase separated and saved. The aqueous phase is extracted with dichloromethane and added to the stored organic phase. The united organic phases are dried, th solvent distilled oof in vacuo and the only residue chromatographed on silica gel with toluene/ethylacetate (l9/l). The titel compond is a yellow, sticky oil.

Analogous to the Examples l and 2, the following compounds of Formula I are obtained.

| Ex. | $R_1$ | $R_2$ | $R_3$ | $R_4$ | $R_5$ | Conf. | phys.chem.charac. |
|---|---|---|---|---|---|---|---|
| 3 | 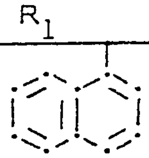 | H | H | $CH_3$ | 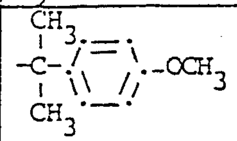 | E | brown yellow oil |
| 4 | -"- | H | H | $CH_3$ | -"- | Z | " " " |
| 5 | -"- | H | H | $CH_3$ | $-CH_2.C_6H_5$ | E | yellow oil |
| 6 | -"- | H | H | $CH_3$ | -"- | Z | " " |
| 7 | -"- | H | H | $CH_3$ | 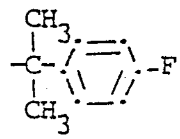 | E | " " |
| 8 | -"- | H | H | $CH_3$ | 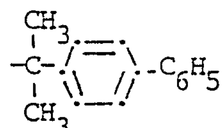 | E | light yellow oil |
| 9 | -"- | H | H | $CH_3$ | 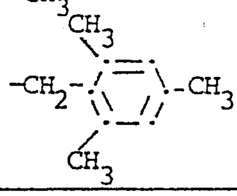 | E | yellow oil |

In the following Table NMR data given. Data comprises peaks in ppm relative to TMS as standard in $CDCL_3$

Types of peaks are

m = multiplet
dt = double triplet
dm = double multiplet
s = singlet
d = doublet
dd = double doublet

NMR-Spectra

| Ex. | Spectrum |
|---|---|
| 1(E) | 8.2-8.35 (m, 1H); 7.65-7.9 (m, 2H); 7.1-7.6 (m, 9H); 6.24 (dt, J= 16 and 2x6.5 Hz, 1 olef.H); 5.75 (dm, J = 16 Hz, 1 olef. H); 3.9 (s, 2H); 3.15 (dd, J = 6.5 and 1 Hz, 2H); 2.23 (s, 3H, N-C$\underline{H}_3$); 1.6 (s, 6H). |
| 1(Z) | 8.2-8.35 (m, 1H); 7.7-7.9 (m, 2H); 7.1-7.65 (m, 9H); 6.12 (dt, J = 11 and 2x7 Hz, 1 olef.H); 5.73 (dm, J = 11 Hz, 1 olef.H); 3.93 (s, 2H); 3.42 (dd, J = 7 and 1 Hz, 2H); 2.26 (s, 3H, N-C$\underline{H}_3$); 1.61 (d, J = 2.5 Hz, 6H). |
| 2 | 8.2-8.4 (m, 1H); 7.7-7.95 (m, 2H); 7.2-7.6 (m, 8H); 6.24 (dt, J = 16 and 2x7 Hz, 1 olef.H); 5.73 (dm, J = 16 Hz, 1 olef.H); 3.91 (s, 2H); 3.15 (dd, J = 7 and 1 Hz, 2H); 2.24 (s, 3H, N-C$\underline{H}_3$); 1.57 (s, 6H). |
| 3 | 8.25-8.4 (m, 1H); 7.7-7.9 (m, 2H); 7.4-7.6 (m, 6H); 6.8-7.0 (m, 2H); 6.26 (dt, J = 16 and 2x6.5 Hz, 1 olef.H); 5.75 (dm, J = 16 Hz, 1 olef.H); 3.91 (s, 2H); 3.8 (s, 3H, O-C$\underline{H}_3$); 3.15 (dd, J = 7 and 1 Hz, 2H); 2.24 (s, 3H, N-C$\underline{H}_3$); 1.58 (s, 6H). |
| 4 | 8.2-8.35 (m, 1H); 7.7-7.9 (m, 2H); 7.35-7.6 (m, 6H); 6.8-6.95 (m, 2H); 6.1 (dt, J = 11 and 2x7 Hz, 1 olef.H); 5.73 (dm, J = 11, 1 olef.H); 3.93 (s, 2H); 3.8 (s, 3H, O-C$\underline{H}_3$); 3.42 (dd, J = 7 and 1 Hz, 2H); 2.26 (s, 3H, N-C$\underline{H}_3$); 1.6 (s, 6H). |
| 5 | 8.2-8.35 (m, 2H); 7.7-7.95 (m, 2H); 7.2-7.65 (m, 9H); 6.28 (dt, J = 16 and 2x7 Hz, 1 olef.H); 5.72 (dm, J = 16 Hz, 1 olef.H); 3.9 (s, 2H); 3.73 (d, J = 2 Hz, 2H); 3.16 (dd, J = 7 and 1 Hz, 2H); 2.23 (s, 3H, N-C$\underline{H}_3$). |

| | |
|---|---|
| 6 | 8.2-8.35 (m, 1H); 7.7-7.95 (m, 2H); 7.2-7.6 (m, 9H); 6.13 (dt, J = 11 and 2x7 Hz, 1 olef.H); 5.7 (dm, J = 11 Hz, 1 olef.H); 3.92 (s, 2H); 3.77 (d, J = 2 Hz, 2H); 3.41 (dd, J = 7 and 1 Hz, 2H); 2.25 (s, 3H, N-$C\underline{H}_3$). |
| 7 | 8.25-8.4 (m, 1H); 7.7-7.95 (m, 2H); 7.4-7.65 (m, 6H); 6.85-7.15 (m, 2H); 6.24 (dt, J = 16 and 2x7 Hz, 1 olef.H); 5.74 (dm, J = 16 Hz, 1 olef.H); 3.9 (s, 2H); 3.14 (dd, J = 7 and 1 Hz, 2H); 2.23 (s, 3H, N-$C\underline{H}_3$); 1.57 (s, 6H). |
| 8 | 8.25-8.4 (m, 1H); 7.3-7.95 (m, 15H); 6.26 (dt, J = 16 and 2x7 Hz, 1 olef.H); 5.76 (dm, J = 16 Hz, 1 olef.H); 3.92 (s, 2H); 3.16 (dd, J = 7 and 1 Hz, 2H); 2.24 (s, 3H, N-$C\underline{H}_3$), 1.64 (s, 6H). |
| 9 | 8.2-8.35 (m, 1H); 7.7-7.95 (m, 2H); 7.35-7.6 (m, 4H); 6.85 (s, 2H); 6.18 (dt, J = 16 and 2x7 Hz, 1 olef.H); 5.6 (dm, J = 16 Hz, 1 olef.H); 3.86 (s, 2H); 3.56 (d, J = 2 Hz, 2H); 3.1 (dd, J = 7 and 0.5 Hz, 2H); 2.35 (s, 3H, N-$C\underline{H}_3$); 2.18 (s, 3H). |

A) l-Bromo-6-methyl-6-phenyl-2-heptene-4-yne

a) 6-Methyl-6-phenyl-l-hepten-4-yne-3-ol.

To a solution of 2.l5 g of 3-methyl-3-phenyl-l-butyne in 3O ml of diethylether are added, at -5O°, under a blanket of Argon, lO ml n-butyl-lithium (l.5 molar) in hexane. After cooling to -7O° O.84 g of acroleine are added dropwise, with stirring. After l hour, the reaction mixture is allowed to reach room temperature then a saturated $NH_4Cl$ solution is carefully added and the mixture extracted with ether. The ether phase is dried and concentrated in vacuo. The purification is effected by column chromatography on silica gel employing initially $CH_2Cl_2$/petrol ether (l/5), to remove unreacted starting material, and then pure $CH_2Cl_2$ as eluant. A yellowish oil is obtained (Rf = O.4 in $CH_2Cl_2$).

b) l-Bromo-6-methyl-6-phenyl-2-hepten-4-yne.

To l g of 6-methyl-6-phenyl-l-hepten-4-yn-3-ol are added at O° a mixture of 2 ml of fuming HBr and l ml of 48 % HBr. Then some drops of ethanol are added and the mixture is stirred for 2 hours at 5°. The reaction mixture is poured into ice water and extracted with ether. The ether phase is washed with aqueous $NaHCO_3$ solution, dried and concentrated in vacuo. The NMR-spectrum of the crude oil shows that it consists of a 3/l mixture of the trans- and cis-l-bromo-6-methyl-6-phenyl-2-hepten-4-yne; it can be employed, without further purification, in the subsequent alkylation reaction.

$^1$H-NMR: 7.O5-7.5 (m, 5 aromat.H); 5.5-6.4 (m, 2 olef.H);[4.l (d, J=8Hz) and 3.9 (d, J=8Hz) in the ratio of l:3, 2H, CH-$C\underline{H}_2$Br]; l.58 (s, 6H).

The following compounds of formula V are obtained analogous to the process of Example A.

| | $R_5$ | A | |
|---|---|---|---|
| B | 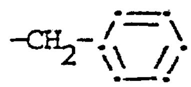 | Br | oil |
| C | 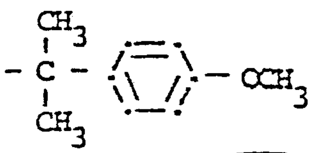 | Br | oil |

## Claims

1. Novel allylamines of formula I

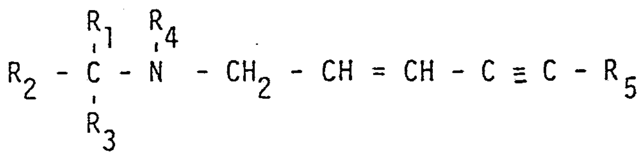

I

wherein $R_1$ is one of the groups of the formula IIa, IIb and IIc,

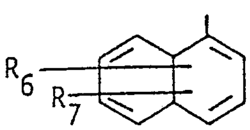

IIa

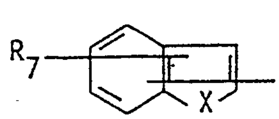

IIb

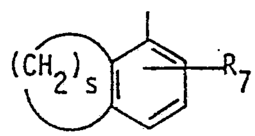

IIc

in which $R_6$ and $R_7$ independently are H, halogen, $CF_3$, $C_{1-5}$alkyl or $C_{1-5}$alkoxy,
X is O, S, O-$CH_2$-, S-$CH_2$-, $CH_2$ or $NR_8$, in which $R_8$ is H or $C_{1-5}$alkyl,
s is 3, 4 or 5,
$R_2$ is H or $C_{1-5}$alkyl,
either $R_3$ and $R_4$ are independently H or $C_{1-5}$alkyl,
or $R_3$ and $R_4$ together $(CH_2)_u$
in which u is 3, 4 or 5,
and $R_5$ is a group of formula IIIa or IIIb

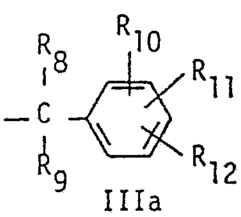

IIIa

or

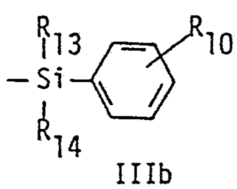

IIIb

wherein $R_8$ and $R_9$ independently are H, $C_{1-5}$alkyl, $C_{1-5}$alkoxy or $CF_3$,
$R_{10}$ $R_{11}$ and $R_{12}$ independently are H, $C_{1-5}$alkyl, $C_{1-5}$alkoxy, $CF_3$, halogen or phenyl, $R_{13}$ and $R_{14}$ independently are $C_{1-5}$alkyl,
in free form or acid addition salt form.

2. Compounds of Claim I, wherein
$R_1$ is the group of formula IIa,
$R_2$ and $R_3$ are H,
$R_4$ is $C_{1-4}$alkyl, and
$R_5$ is the group of formula IIIa,
in which $R_8$ and $R_9$ are H or $C_{1-4}$alkyl.

3. The compound of Claim 2, wherein $R_8$ and $R_9$ are $C_{1-4}$alkyl.

4. The compound of Claim 2, wherein $R_8$ and $R_9$ are $CH_3$.

5. The compound of any of claims I to 4, wherein $R_{10}$ is H, halogen, $C_{1-4}$alkoxy, $_{1-4}$alkyl or phenyl.

6. The compound of Claim 5, wherein $R_{10}$ is in the para-position, $R_{11}$ and $R_{12}$ are selected from H and $C_{1-4}$alkyl and $R_6$ and $R_7$ are hydrogen.

7. (E)-N-Methyl-N-(I-naphthylmethyl)-6-methyl-6-phenyl-2-hepten-4-ynamine.

8. (E)-N-Methyl-N-(I-naphthylmethyl)-6-methyl-6-(4-fluorophenyl)-2-hepten-4-ynamine.

9. A plant fungicidal composition comprising a compound according to Claims I to 8 in free form or in agriculturally acceptable acid addition salt, alcoholate or metal complex form and an agriculturally acceptable diluent.

IO. A method of combatting phytopathogenic fungi which comprises applying to the locus thereof a fungicidally effective amount of a compound claimed in any one of Claims I to 8 in free form or in agriculturally acceptable acid addition salt, alcoholate or metal complex form.

II. A pharmaceutical composition which comprises a compound according to any one of Claims I to 8 in free form or in a chemotherapeutically acceptable acid addition salt form thereof and a chemotherapeutically acceptable diluent or carrier.

I2. A compound according to any one of Claims I to 8 in free form or in a chemotherapeutically acceptable acid addition salt form for use as a pharmaceutical.

13. Process of preparing a compound of formula I, stated in Claim I, in free base form or in acid addition salt form, which comprises

a) reacting a compound of formula IV

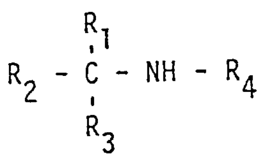

IV.

wherein $R_1$ to $R_4$ are defined in Claim I,
with a compound of formula V

$A - CH_2 - CH = CH - C \equiv C - R_5$ V

wherein $R_5$ is as defined in Claim I, and A is a leaving group,

or b) obtaining compounds of formula Ia

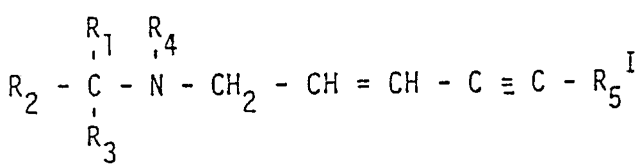

Ia

wherein $R_1$ to $R_4$ as defined in Claim I, and $R_5^I$ is a group of formula IIIa as defined in Claim I, by substituting in a compound of formula VI

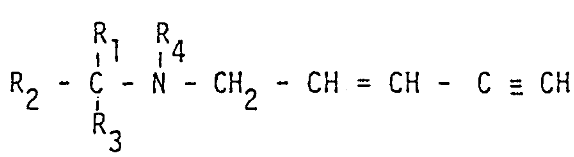

VI

wherein $R_1$ to $R_4$ are as defined in Claim I,
the acetylenic hydrogen by an alkali metal and reacting the thus obtained alkali metal salt of the compound of formula VI with a compound of formula IIIc

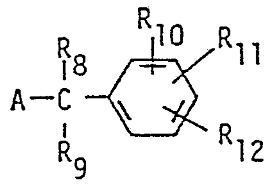 IIIc

wherein $R_8$ to $R_{12}$ and A are as defined in Claim I,
and recovering the thus obtained compound of formula I in free form or in the form of an acid addition salt form.

European Patent Office

# EUROPEAN SEARCH REPORT

Application number

EP 87 81 0381

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,X | DE-A-3 442 529 (SANDOZ-PATENT)<br>* Claims; page 8 * | 1,9-13 | C 07 C 87/29<br>C 07 C 87/457<br>C 07 C 93/14<br>C 07 D 307/81<br>C 07 D 333/58<br>C 07 F 7/08<br>A 61 K 31/135<br>A 61 K 31/33<br>A 01 N 33/04<br>A 01 N 43/00 |
| Y | GB-A-2 091 747 (SANDOZ)<br>* Claims * | 1,9-13 | |
| Y | FR-A-2 526 423 (SANDOZ)<br>* Claims * | 1,9-13 | |
| Y | DE-A-3 405 334 (SANDOZ-PATENT)<br>* Claims; pages 7,8 * | 1,9-13 | |
| Y | EP-A-0 000 896 (SANDOZ)<br>* Claims * | 1,9-13 | |
| D,X | EP-A-0 024 587 (SANDOZ)<br>* Claims * | 1,9-13 | TECHNICAL FIELDS SEARCHED (Int. Cl.4)<br>C 07 C 87/00<br>C 07 C 93/00<br>C 07 D 307/00<br>C 07 D 333/00<br>C 07 F 7/00<br>A 61 K 31/00<br>A 01 N 31/00<br>A 01 N 43/00 |

The present search report has been drawn up for all claims

| Place of search | Date of completion of the search | Examiner |
|---|---|---|
| THE HAGUE | 20-10-1987 | MOREAU J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document